# EUROPÄISCHE PATENTANMELDUNG

(11) **EP 1 156 441 A2**
(43) Veröffentlichungstag der Anmeldung: **21.11.2001**
(21) Anmeldenummer: 01111666.2
(22) Anmeldetag: 14.05.2001
(51) Int. Cl.: G06F 19/00

(54) **Medizinische Daten erfassendes System**

(30) Priorität: 16.05.2000 DE 10023860
(71) Anmelder: LMB Technologie GmbH, 85445 Schwaig (DE)
(72) Erfinder: Jentsch, Klaus, 85445 Schwaig (DE)
(74) Vertreter: Winter, Brandl, Fürniss, Hübner, Röss, Kaiser, Polte Partnerschaft

(57) **Zusammenfassung**

Ein erfindungsgemäßes medizinische Daten erfassendes System weist mindestens eine erste Sende/Empfangseinrichtung (1), die mit einer Datenverarbeitungseinrichtung (3) verbunden ist; und mindestens eine zweite Sende/Empfangseinrichtung (2) auf, die mit einer medizinische Daten liefernden Einrichtung (4) verbunden ist. Medizinische Daten werden drahtlos von der medizinische Daten liefernden Einrichtung (4) über die mindestens eine erste und die mindestens eine zweite Sende/Empfangseinrichtung (1, 2) zu der Datenverarbeitungseinrichtung (3) gesendet. In Abhängigkeit von einem spezifischen Typ der medizinische Daten liefernden Einrichtung (4) werden die mindestens eine erste und die mindestens eine zweite Sende/Empfangseinrichtung (1, 2) derart konfiguriert, daß für den spezifischen Typ der medizinische Daten liefernden Einrichtung (4) spezifische Daten zu der Datenverarbeitungseinrichtung (3) gesendet werden.

## Beschreibung

Die vorliegende Erfindung betrifft ein medizinische Daten erfassendes System, bei welchem Daten von einer medizinische Daten liefernden Einrichtung zu einer Datenverarbeitungseinrichtung gesendet werden.

Im Stand der Technik ist es bekannt, daß Hersteller verschiedener medizintechnischer Geräte, wie zum Beispiel Zentrifugen, Plasmapherese-Spendeplätzen usw., Möglichkeiten anbieten, von derartigen medizintechnischen Geräten erzielte medizinische Daten drahtgebunden und/oder drahtlos zu einer Datenverarbeitungseinrichtung zu senden, bei der sie weiterverarbeitet und/oder gespeichert werden können. Insbesondere die drahtlose Übertragung von Daten ist hier von Vorteil, da auf eine Verkabelung verzichtet werden kann.

Jedoch weisen diese bisherigen Möglichkeiten, ein medizinische Daten erfassendes System zu schaffen, erhebliche Nachteile auf. Zum Beispiel muß für jedes medizintechnische Gerät bei jedem Hersteller eine zusätzliche Ausstattung erworben werden, die jedoch nur für das jeweilige medizintechnische Gerät des Herstellers verwendet werden kann. Weiterhin gibt es bis heute keinen Standard für eine derartige Datenübertragung zwischen medizintechnischen Geräten und Datenverarbeitungseinrichtungen, so daß es zu Inkompatibilitäten kommen kann, wenn Daten von mehreren medizintechnischen Geräten zum Beispiel strukturiert in einer einzigen Datenbank abgelegt werden sollen.

Es ist demgemäß die Aufgabe der vorliegenden Erfindung, ein medizinische Daten erfassendes System zu schaffen, mittels welchem beliebige medizinische Daten liefernde Einrichtungen einfach und zuverlässig mit einer Datenverarbeitungseinrichtung kommunizieren können.

Diese Aufgabe wird mit den in Anspruch 1 angegebenen Maßnahmen gelöst.

Genauer gesagt weist ein erfindungsgemäßes medizinische Daten erfassendes System mindestens eine erste Sende/Empfangseinrichtung, die mit einer Datenverarbeitungseinrichtung verbunden ist; und mindestens eine zweite Sende/Empfangseinrichtung auf, die mit einer medizinische Daten liefernden Einrichtung verbunden ist. Medizinische Daten werden drahtlos von der medizinische Daten liefernden Einrichtung über die mindestens eine erste und die mindestens eine zweite Sende/Empfangseinrichtung zu der Datenverarbeitungseinrichtung gesendet. In Abhängigkeit von einem spezifischen Typ der medizinische Daten liefernden Einrichtung werden die mindestens eine erste und die mindestens eine zweite Sende/Empfangseinrichtung derart konfiguriert, daß für den spezifischen Typ der medizinische Daten liefernden Einrichtung spezifische Daten zu der Datenverarbeitungseinrichtung gesendet werden.

Erfindungsgemäß ist es erkannt worden, daß es vorteilhaft ist, die mindestens eine erste und die mindestens eine zweite Sende/Empfangsvorrichtung derart zu konfigurieren, daß sie an einen jeweiligen spezifischen Typ einer medizinische Daten liefernden Einrichtung angepaßt werden.

Durch diese Maßnahme ist es möglich, für verschiedene Typen von medizinische Daten liefernden Einrichtungen immer gleiche Typen der ersten und zweiten Sende/Empfangseinrichtung zu verwenden, die an den jeweiligen Verwendungszweck angepaßt werden. Dadurch wird ein einfaches und zuverlässiges medizinische Daten erfassendes System geschaffen, welches weiterhin äußerst flexibel einsetzbar ist. Die flexible Einsetzbarkeit des medizinische Daten erfassenden Systems ist unter anderem durch die Möglichkeit der Umkonfiguration des Systems für unterschiedliche Anwendungszwecke gegeben, ohne daß bei einer derartigen Umkonfiguration außer einer neuen medizinische Daten liefernden Einrichtung weitere neue Komponenten erforderlich sind.

Wenn zum Beispiel eine weitere medizinische Daten liefernde Einrichtung mit dem medizinische Daten erfassenden System zu verbinden ist, wird lediglich eine weitere standardisierte zweite Sende/Empfangseinrichtung mit der weiteren medizinische Daten liefernden Einrichtung verbunden und werden die weitere zweite Sende/Empfangseinrichtung und die erste Sende/Empfangseinrichtung derart konfiguriert, daß für den spezifischen Typ der weiteren medizinische Daten liefernden Einrichtung ebenso spezifische Daten zu der Datenverarbeitungseinrichtung gesendet werden.

Die vorliegende Erfindung weist dahingehend erhebliche Vorteile auf, daß es möglich ist, Inkompatibilitätsprobleme dadurch zu vermeiden, daß alle mit dem medizinische Daten erfassenden System verbundenen medizinische Daten liefernden Einrichtungen an das medizinische Daten erfassende System angepaßt werden, ohne daß eine zusätzliche Software- und/oder Hardwareausstattung eines Herstellers einer spezifischen medizinischen Daten liefernden Einrichtung erforderlich ist, die nicht für andere Typen von medizinische Daten liefernden Einrichtungen verwendbar ist. Dadurch ist es möglich, für alle medizinische Daten liefernden Einrichtungen gleiche Übertragungs-, Verarbeitungs- und/oder Sicherheitsstandards zu verwenden, auch wenn die zu verarbeitenden Daten unterschiedliche Inhalte aufweisen.

Gemäß einer Ausgestaltung der vorliegenden Erfindung können die mindestens eine erste Sende/Empfangseinrichtung und die mindestens eine zweite Sende/Empfangseinrichtung bidirektional miteinander kommunizieren.

Gemäß einer weiteren Ausgestaltung der vorliegenden Erfindung sind die mindestens eine erste und die mindestens eine zweite Sende/Empfangseinrichtung mittels eines auf der Datenverarbeitungseinrichtung vorgesehenen Programms konfigurierbar.

Gemäß einer weiteren Ausgestaltung der vorliegenden Erfindung können die zu der Datenverarbeitungseinrichtung gesendeten spezifischen Daten auf dieser gespeichert werden.

Gemäß einer weiteren Ausgestaltung der vorliegenden Erfindung sind die spezifischen Daten mittels der Datenverarbeitungseinrichtung verarbeitbar.

Gemäß einer weiteren Ausgestaltung der vorliegenden Erfindung ist die mindestens eine erste Sende/Empfangseinrichtung mittels einer RS232-Schnittstelle oder einer USB-Schnittstelle mit der Datenverarbeitungseinrichtung verbunden.

Gemäß einer weiteren Ausgestaltung der vorliegenden Erfindung ist die mindestens eine zweite Sende/Empfangseinrichtung mittels einer RS232-Schnittstelle oder einer USB-Schnittstelle mit der medizinische Daten liefernden Einrichtung verbunden.

Gemäß einer weiteren Ausgestaltung der vorliegenden Erfindung erfolgt das Senden der spezifischen Daten per Funk.

Gemäß einer weiteren Ausgestaltung der vorliegenden Erfindung weist das System mehrere erste und/oder zweite Sende/Empfangseinrichtungen auf, wobei an jede jeweilige zweite Sende/Empfangseinrichtung eine jeweilige medizinische Daten liefernde Einrichtung beliebigen Typs anschließbar ist.

Gemäß einer weiteren Ausgestaltung der vorliegenden Erfindung ist an die Datenverarbeitungsvorrichtung ferner ein Scanner, eine Tastatur und/oder ein Strichcode-Leser angeschlossen.

Weitere vorteilhafte Ausgestaltungen der vorliegenden Erfindung sind Gegenstand der abhängigen Ansprüche.

Die vorliegende Erfindung wird nachstehend anhand von Ausführungsbeispielen unter Bezugnahme auf die beiliegende Zeichnung näher erläutert.

Es zeigt:
- **Fig. 1**: eine schematische Darstellung eines medizinische Daten erfassenden Systems gemäß einem ersten Ausführungsbeispiel der vorliegenden Erfindung;
- **Fig. 2**: eine schematische Darstellung eines medizinische Daten erfassenden Systems gemäß einem zweiten Ausführungsbeispiel der vorliegenden Erfindung; und
- **Fig. 3**: eine schematische Darstellung eines medizinische Daten erfassenden Systems gemäß einem dritten Ausführungsbeispiel der vorliegenden Erfindung.

Nachstehend erfolgt die Beschreibung eines ersten Ausführungsbeispiels der vorliegenden Erfindung.

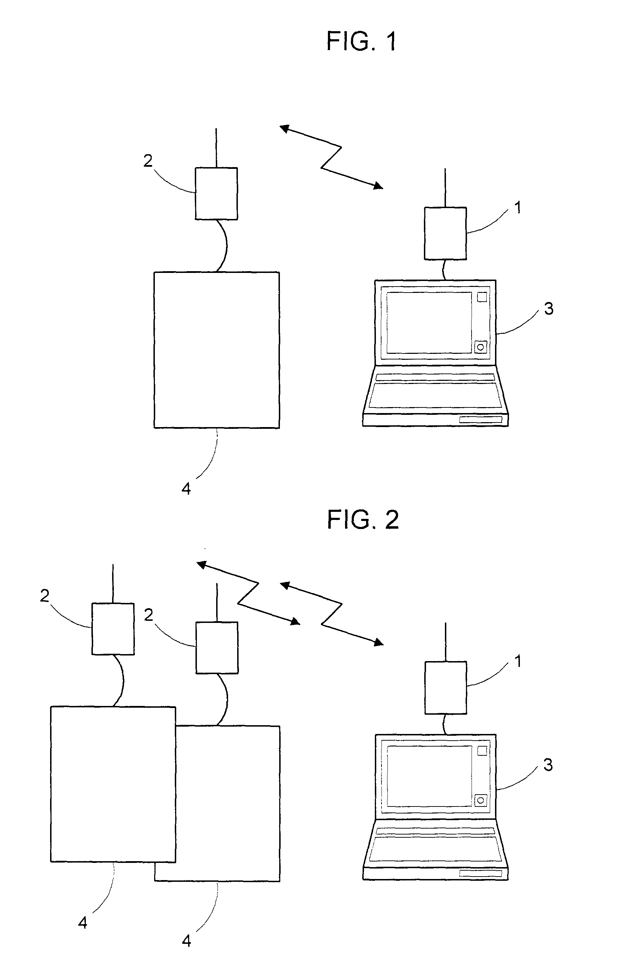

Fig. 1 zeigt eine schematische Darstellung eines medizinische Daten erfassenden Systems gemäß dem ersten Ausführungsbeispiel der vorliegenden Erfindung.

In Fig. 1 bezeichnet das Bezugszeichen 1 eine erste Sende/Empfangseinrichtung, bezeichnet das Bezugszeichen 2 eine zweite Sende/Empfangseinrichtung, bezeichnet das Bezugszeichen 3 eine Datenverarbeitungseinrichtung und bezeichnet das Bezugszeichen 4 eine medizinische Daten liefernde Einrichtung.

Die Datenverarbeitungseinrichtung 3 kann zum Beispiel ein Rechner, wie zum Beispiel ein Notebook oder ein mit einem Netzwerk verbundener Desktop-Computer, sein. Ferner können an die Datenverarbeitungseinrichtung 3 weitere Einrichtungen, wie zum Beispiel ein Scanner, eine Tastatur und/oder ein Strichcode-Leser angeschlossen sein, die zum Beispiel dazu dienen, weitere Informationen aufnehmen zu können.

Die medizinische Daten liefernde Einrichtung 4 ist eine beliebige Einrichtung, von der medizinische Daten zu erzielen sind, wie zum Beispiel eine Zentrifuge, ein Hämoglobin-Meßplatz, ein Plasmapherese-Spendeplatz. eine Plasmapresse oder jede beliebige Art von medizintechnischen Laborgeräten, wie es aus den nachstehenden Ausführungen deutlich wird.

Die erste Sende/Empfangseinrichtung 1 ist über eine geeignete Verbindung, wie zum Beispiel eine RS232-Schnittstelle, eine USB- bzw. Universal-Serial-Bus-Schnittstelle usw., mit der Datenverarbeitungseinrichtung 3 verbunden. Alternativ besteht ebenso die Möglichkeit, eine drahtlose Verbindung, wie zum Beispiel über eine Infrarot-Schnittstelle, vorzusehen. Die zweite Sende/Empfangseinrichtung 2 ist auf eine ähnliche Weise mit der medizinische Daten liefernden Einrichtung 4 verbunden. Die erste und die zweite Sende/Empfangseinrichtung 1 bzw. 2 sind derart ausgelegt, daß sie miteinander eine bidirektionale Kommunikation ausführen können.

Nachstehend erfolgt die Beschreibung der Funktionsweise des medizinische Daten liefernden Systems gemäß dem ersten Ausführungsbeispiel der vorliegenden Erfindung.

Wenn eine medizinische Daten liefernde Einrichtung 4 mit der Datenverarbeitungseinrichtung 3 zu verbinden ist, um medizinische Daten von der medizinische Daten liefernden Einrichtung 4 zu der Datenverarbeitungseinrichtung 3 zu übertragen und mittels dieser zu verarbeiten, wird die zweite Sende/Empfangseinrichtung 2 mit der medizinische Daten liefernden Einrichtung 4 verbunden und wird die erste Sende/Empfangseinrichtung 1 mit der Datenverarbeitungseinrichtung 3, sofern dies nicht schon der Fall ist.

Danach wird auf der Datenverarbeitungseinrichtung 3 ein Installationsprogramm aufgerufen, mittels dem die medizinische Daten liefernde Einrichtung 4 ähnlich einem Drucker oder dergleichen unter Windows installierbar ist. Das bedeutet, die erste und die zweite Sende/Empfangseinrichtung 1 bzw. 2 werden hinsichtlich ihres Betriebs derart angepaßt, daß sie abhängig von einem spezifischen Typ der medizinische Daten liefernden Einrichtung 4 eine bidirektionale Kommunikation durchführen können, um medizinische Daten von der medizinische Daten liefernden Einrichtung 4 zu der Datenverarbeitungseinrichtung 3 übertragen. Die Datenverarbeitungseinrichtung 3 führt im Anschluß an ein derartiges Übertragen von Daten eine Verarbeitung dieser Daten durch. Eine derartige Verarbeitung kann zum Beispiel ein Aufbereiten, Auswerten, Speichern und/oder Weiterleiten der übertragenen Daten sein.

Hierbei ist es vorteilhaft, daß bei einem Wechsel der medizinische Daten liefernden Einrichtung von einem Typ zu einem anderen Typ, eine einfache Anpaßbarkeit des medizinische Daten liefernden Systems dadurch erzielt wird, daß lediglich erneut das Installationsprogramm ausgeführt werden muß, um den neuen Typ des medizinische Daten liefernden Systems zu installieren.

Angewendet kann ein derartiges medizinische Daten erfassendes System zum Beispiel in der Blutbank-Technik, in der Pharmazie, im Labor, in der Lagerlogistik, im Transport usw., werden.

Insbesondere ist anzumerken, daß es bei dem zuvor beschriebenen medizinische Daten erfassenden System wesentlich ist, daß die erste und zweite Sende/Empfangseinrichtung 1 bzw. 2 die medizinische Daten liefernde Einrichtung 4 mittels der Datenverarbeitungseinrichtung 3 derart konfigurieren können, daß die Konfiguration an jeweilige Schnittstellen jeweiliger medizinischer Daten liefernder Einrichtungen 4, deren Übertragungstandards, die Art der von der zweiten Sende/Empfangseinrichtung 2 zu der ersten Sende/Empfangseinrichtung 1 zu übertragenden Daten, usw. anpaßt.

Nachstehend erfolgt die Beschreibung eines zweiten Ausführungsbeispiels der vorliegenden Erfindung.

Fig. 2 zeigt eine schematische Darstellung eines medizinische Daten erfassenden Systems gemäß dem zweiten Ausführungsbeispiel der vorliegenden Erfindung.

In Fig. 2 bezeichnen die gleichen Bezugszeichen wie in Fig. 1 die gleichen oder entsprechende Teile.

Das zweite Ausführungsbeispiel der vorliegenden Erfindung ist ausgenommen des nachstehend beschriebenen Unterschieds zu dem ersten Ausführungsbeispiel der vorliegenden Erfindung gleich, weshalb eine redundante Beschreibung gleicher Teile und Funktionsweisen an dieser Stelle weggelassen wird.

Wie es in Fig. 2 gezeigt ist, weist das zweite Ausführungsbeispiel der vorliegenden Erfindung zwei zweite Sende/Empfangseinrichtungen 3 auf. Es können jedoch eine beliebige Anzahl von zweiten Sende/Empfangseinrichtungen 3 vorgesehen sein. Jede zweite Sende/Empfangseinrichtung 3 ist mit einer medizinische Daten liefernden Einrichtung 4 eines spezifischen Typs verbunden, wobei die Typen beliebig sein können.

Gemäß dem zweiten Ausführungsbeispiel der vorliegenden Erfindung kann das erste Ausführungsbeispiel der vorliegenden Erfindung derart abgeändert werden, daß mehrere medizinische Daten liefernde Einrichtungen 4 derart mittels der Datenverarbeitungseinrichtung 3 konfiguriert werden können, daß von einer jeweiligen medizinische Daten liefernden Einrichtung 4 über jeweilige zweite Sende/Empfangsvorrichtungen 2 und die erste Sende/Empfangseinrichtung 1 Daten zu der Datenverarbeitungseinrichtung 3 übertragen und mittels dieser verarbeitet werden können.

Dies bedeutet, daß die erste Sende/Empfangseinrichtung 1 imstande ist, Daten von mehreren medizinische Daten liefernden Einrichtungen 4 aufzunehmen. Die Daten werden dabei je nach Herkunft von einer bestimmten medizinische Daten liefernden Einrichtung 4 konvertiert und strukturiert abgespeichert und/oder verarbeitet, wie dies in ähnlicher Weise bei dem ersten Ausführungsbeispiel der vorliegenden Erfindung der Fall ist.

Es ist anzumerken, daß sowohl bei dem ersten Ausführungsbeispiel der vorliegenden Erfindung als auch dem zweiten Ausführungsbeispiel der vorliegenden Erfindung keine herkömmlichen Computerstandards verwendet werden, da derartige Standards hinsichtlich der zuvor beschriebenen Anwendung ungeeignet sind. Vielmehr werden speziell entwickelte Standards verwendet, die zur Erhöhung der Sicherheit beitragen.

Nachstehend erfolgt die Beschreibung eines dritten Ausführungsbeispiels der vorliegenden Erfindung.

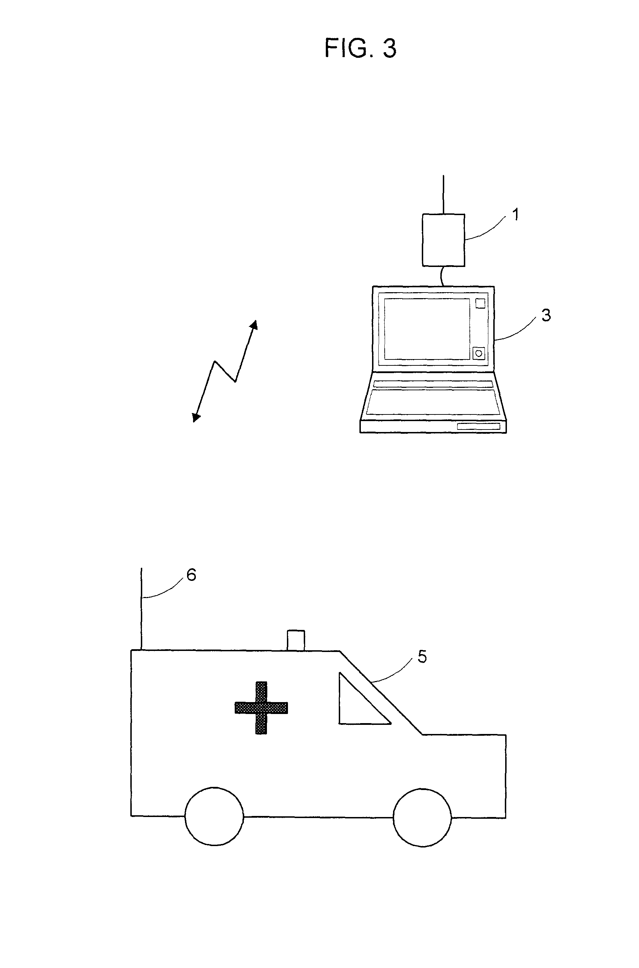

Fig. 3 zeigt eine schematische Darstellung eines medizinische Daten erfassenden Systems gemäß dem dritten Ausführungsbeispiel der vorliegenden Erfindung.

Das dritte Ausführungsbeispiel der vorliegenden Erfindung ist ausgenommen des nachstehend beschriebenen Unterschieds zu dem ersten oder zweiten Ausführungsbeispiel der vorliegenden Erfindung gleich, weshalb eine redundante Beschreibung gleicher Teile und Funktionsweisen an dieser Stelle weggelassen wird.

Das dritte Ausführungsbeispiel der vorliegenden Erfindung ist eine Ausgestaltung des ersten oder zweiten Ausführungsbeispiels der vorliegenden Erfindung und stellt eine Anwendung dieser Ausführungsbeispiele der vorliegenden Erfindung bei einem Kühltransport von zum Beispiel Blutkonserven oder dergleichen dar.

In Fig. 3 bezeichnet weiterhin das Bezugszeichen 5 einen Kühltransporter und bezeichnet das Bezugszeichen 6 eine Antenne.

Die Antenne 6 kann mit einer oder mehreren zweiten Sende/Empfangseinrichtungen 2 in Verbindung stehen, die in dem Kühltransporter 5 vorgesehen sind, um Daten zu einer extern vorhandenen ersten Sende/Empfangseinrichtung 1 und letztendlich zu einer Datenverarbeitungseinrichtung 3 zu übertragen. Die weitere Funktionsweise ist zu der des ersten oder zweiten Ausführungsbeispiels der vorliegenden Erfindung gleich.

Schließlich ist anzumerken, daß bei allen zuvor beschriebenen Ausführungsbeispielen ebenso mehrere erste Sende/Empfangseinrichtungen 1 vorgesehen sein können, die Daten von einer oder mehreren zweiten Sende/Empfangseinrichtungen 2 aufnehmen und schließlich der Datenverarbeitungseinrichtung 3 zuführen. Auch können mehrere Datenverarbeitungseinrichtungen 3 gleichzeitig vorgesehen werden.

Nachstehend erfolgt die Beschreibung von Anwendungen der zuvor beschriebenen Ausführungsbeispiele der vorliegenden Erfindung.

Eine erste Anwendung besteht darin, das medizinische Daten erfassende System mit einer oder mehreren Zentrifugen zu verwenden, wie sie zum Beispiel von den Firmen Hettich, Heräeus und Jouan auf dem Markt vertrieben werden. Bei dieser ersten Anwendung können Daten, wie zum Beispiel Anwendernummer, Konservennummer, Startzeit, Drehzahl, Profil, Stoppzeit, Seriennummer usw., mittels des medizinische Daten erfassenden Systems übertragen und verarbeitet werden.

Eine zweite Anwendung bezieht sich auf das dritte Ausführungsbeispiel der vorliegenden Erfindung. Bei einer derartigen Anwendung bei einem Kühltransport können Daten, wie zum Beispiel Kfz-Nummer, Fahrerkennung. Konservennummer, Einlagerungszeit, Transportstart, Transportende, Temperaturkurve, usw., mittels des medizinische Daten erfassenden Systems übertragen und verarbeitet werden.

Eine dritte Anwendung besteht darin, daß medizinische Daten erfassende System mit einem oder mehreren Hämoglobin-Meßplätzen zu verwenden. Bei dieser dritten Anwendung können Daten, wie zum Beispiel Operatornummer, HB-Wert, Konservennummer, Seriennummer, Uhrzeit, usw., mittels des medizinische Daten erfassenden Systems übertragen und verarbeitet werden.

Eine vierte Anwendung besteht darin, daß medizinische Daten erfassende System mit einem oder mehreren Plasmapherese-Spendeplätzen zu verwenden, wie sie zum Beispiel von den Firmen Baxter, Fresenius, Cobe und Haemonetics auf dem Markt vertrieben werden. Bei dieser vierten Anwendung können Daten, wie zum Beispiel Anwendernummer, Lotnummer-Beutel, Programmtyp, Konservennummer, Spendernummer, Röhrchennummer, Fehlercode, Uhrzeit, usw., mittels des medizinische Daten erfassenden Systems übertragen und verarbeitet werden.

Eine fünfte Anwendung besteht darin, daß medizinische Daten erfassende System mit einem oder mehreren Plasmapressen zu verwenden. Bei dieser fünften Anwendung können Daten, wie zum Beispiel Anwendernummer, Konservennummer, Startzeit, Stoppzeit, Programmwahl, usw., mittels des medizinische Daten erfassenden Systems übertragen und verarbeitet werden.

Eine sechste Anwendung besteht darin, daß medizinische Daten erfassende System mit einem oder mehreren Laborgeräten unterschiedlicher oder gleicher Typen zu verwenden. Bei dieser sechsten Anwendung können Daten, wie zum Beispiel Blutgruppe, Rhesusfaktor, Hämoglobin, Kell, Startzeit, Benutzerdaten, usw., mittels des medizinische Daten erfassenden Systems übertragen und verarbeitet werden.

Ferner sind auch beliebige Kombinationen der zuvor beschriebenen Anwendungen möglich.

Wie es aus den vorhergehenden Ausführungen ersichtlich ist, ist die vorliegende Erfindung in vielen Gebieten, wie zum Beispiel in der Blutbank-Technik, in der Pharmazie, im Labor, in der Lagerlogistik, im Transport, usw. anwendbar und kann überall dort eingesetzt werden, wo es darum geht, medizinische Daten, die sich auf medizintechnische Produkte oder ähnliches beziehen, zu erfassen und zu verarbeiten.

Bezüglich weiterer Merkmale und Vorteile der vorliegenden Erfindung wird ausdrücklich auf die Offenbarung der Zeichnung verwiesen.

## Patentansprüche

1. Medizinische Daten erfassendes System, das aufweist:
mindestens eine erste Sende/Empfangseinrichtung, die mit einer Datenverarbeitungseinrichtung verbunden ist; und
mindestens eine zweite Sende/Empfangseinrichtung auf, die mit einer medizinische Daten liefernden Einrichtung verbunden ist, wobei medizinische Daten drahtlos von der medizinische Daten liefernden Einrichtung über die mindestens eine erste und die mindestens eine zweite Sende/Empfangseinrichtung zu der Datenverarbeitungseinrichtung gesendet werden, wobei
die mindestens eine erste und die mindestens eine zweite Sende/Empfangseinrichtung in Abhängigkeit von einem spezifischen Typ der medizinische Daten liefernden Einrichtung derart konfiguriert werden, daß für den spezifischen Typ der medizinische Daten liefernden Einrichtung spezifische Daten zu der Datenverarbeitungseinrichtung gesendet werden.

2. System nach Anspruch 1, ***dadurch gekennzeichnet, daß*** die mindestens eine erste Sende/Empfangseinrichtung und die mindestens eine zweite Sende/Empfangseinrichtung bidirektional miteinander kommunizieren.

3. System nach Anspruch 1 oder 2, ***dadurch gekennzeichnet, daß*** die mindestens eine erste und die mindestens eine zweite Sende/Empfangseinrichtung mittels eines auf der Datenverarbeitungseinrichtung vorgesehenen Programms konfigurierbar sind.

4. System nach einem der vorhergehenden Ansprüche, ***dadurch gekennzeichnet, daß*** die zu der Datenverarbeitungseinrichtung gesendeten spezifischen Daten auf dieser gespeichert werden.

5. System nach einem der vorhergehenden Ansprüche, ***dadurch gekennzeichnet, daß*** die spezifischen Daten mittels der Datenverarbeitungseinrichtung verarbeitbar sind.

6. System nach einem der vorhergehenden Ansprüche, ***dadurch gekennzeichnet, daß*** die mindestens eine erste Sende/Empfangseinrichtung mittels einer RS232-Schnittstelle oder einer USB-Schnittstelle mit der Datenverarbeitungseinrichtung verbunden ist.

7. System nach einem der vorhergehenden Ansprüche, ***dadurch gekennzeichnet, daß*** die mindestens eine zweite Sende/Empfangseinrichtung mittels einer RS232-Schnittstelle oder einer USB-Schnittstelle mit der medizinische Daten liefernden Einrichtung verbunden ist.

8. System nach einem der vorhergehenden Ansprüche, ***dadurch gekennzeichnet, daß*** das Senden der spezifischen Daten per Funk erfolgt.

9. System nach einem der vorhergehenden Ansprüche, *da* *durch gekennzeichnet, daß* es mehrere erste und/oder zweite Sende/Empfangseinrichtungen aufweist, wobei an jede jeweilige zweite Sende/Empfangseinrichtung eine jeweilige medizinische Daten liefernde Einrichtung beliebigen Typs anschließbar ist.

10. System nach einem der vorhergehenden Ansprüche, ***dadurch gekennzeichnet*, *daß*** an die Datenverarbeitungsvorrichtung ferner ein Scanner, eine Tastatur und/oder ein Strichcode-Leser angeschlossen ist.
